Europäisches Patentamt

European Patent Office

Office européen des brevets

(11) Publication number: **0 415 982 B1**

## (12) EUROPEAN PATENT SPECIFICATION

(45) Date of publication of patent specification: **24.11.93**

(51) Int. Cl.5: **C07C 43/20**, C07C 43/225, C07C 43/23, C07C 69/92, C07C 41/14, C07C 41/16

(21) Application number: **89905891.1**

(22) Date of filing: **24.04.89**

(86) International application number: **PCT/US89/01682**

(87) International publication number: **WO 89/10912 (16.11.89 89/27)**

(54) METHOD OF PREPARING AROMATIC ETHERS FROM IODOAROMATIC COMPOUNDS.

(30) Priority: **02.05.88 US 188990**

(43) Date of publication of application:
**13.03.91 Bulletin 91/11**

(45) Publication of the grant of the patent:
**24.11.93 Bulletin 93/47**

(84) Designated Contracting States:
**AT BE CH DE FR GB IT LI LU NL SE**

(56) References cited:

**Journal of the Chemical Society, Chemical Communications, no. 9, 1983, M. NUMAZAWA et al, "Efficient synthesis of 2-methoxy- and -4-methoxy-estrogens", pages 533-534**

**Journal of the American Society, vol. 96, no. 9, 1 May 1974, American Chemical Society, US; G.M. WHITESIDES et al, "Copper(I) Alkoxides. Synthesis, reactions and thermal decomposition", pages 2829-2835**

(73) Proprietor: **EASTMAN KODAK COMPANY**
**343 State Street**
**Rochester, New York 14650-2201(US)**

(72) Inventor: **LARKINS, Thomas, Hassell, Jr.**
**Route 8, 4408 Beechcliff**
**Kingsport, TN 37664(US)**

(74) Representative: **Parent, Yves et al**
**Kodak-Pathé**
**Département Brevets et Licences**
**Centre de Recherches et de Technologie**
**Zone Industrielle**
**F-71102 Chalon-sur-Saône Cédex (FR)**

Journal of the American Chemical Society, vol. 109, no. 5, 4 March 1987, American Chemical Society, US; A.J. PAINE: "Mechanisms and models for copper mediated nucelophilic aromatic substitution. ": A single catalytic species from three different oxidation states of copper in an Ullmann synthesis of triarylamines", pages 1496-1502

Chemical Abstracts, vol. 79, no. 3, 23 July 1973, Columbus, Ohio, US; TRAN DINH TUONG et al, "Novel catalyst system for the Ullmann condensation in aqueous solution. Reaction of a haloanthraquinone with aniline", page 378, abstract no. 17738a

**Description**

This invention relates to a method of preparing aromatic ethers from iodoaromatic compounds and alcohols wherein a reducing agent is used to minimize the formation of undesirable by-products.

Aromatic compounds containing one or more hydroxy groups attached to the aromatic ring are highly useful chemicals currently produced in large quantities. Examples of such hydroxy aromatic compounds are hydroquinone, resorcinol, catechol, hydroxybenzoic acid and amino-phenols. Manufacture of these compounds using currently available technology is typically quite expensive.

One method presently known in the art to form an aromatic hydroxy compound comprises reacting a halogenated aromatic compound with a strong base, followed by neutralization of the reaction product. This reaction was at one time practiced commercially for the conversion of chlorobenzene to phenol under very extreme conditions of temperature and pressure, using copper as a catalyst in some of these conversions. However, extension of this idea to dihalogenated compounds gave very low yields due to the occurrence of numerous side reactions which took place due to the severe reaction conditions required.

The need exists for a method which can produce high yields of aromatic hydroxy compounds cheaply and efficiently.

Another method to form an aromatic hydroxy compound is represented by Ullman condensations referred to in J. Am. Chem. Soc. 1987, 109, 1496-1502 which are defined as copper catalyzed nucleophilic aromatic substitutions on unactivated aryl halides according to the following scheme :

$$\text{NuH + ArX} \xrightarrow{\text{"Cu"/base}} \text{ArNu + HX}$$

where NuH is inter alia ROH,
X is Cl, Br, I ;
"Cu" is Cu metal, oxides, salts, alloys, complexes, etc.
base is $K_2CO_3$ KOH, etc.

The problem solved by the present invention is to improve this process in order to achieve a reduced amount of undesirable aryl by-products and an increased yield of desired products.

According to the present invention, it has been discovered that the ether-forming reaction of an iodoaryl compound with an alcohol in the presence of a basic compound and a copper catalyst can be conducted with minimal formation of undesirable aryl by-products and high yield of desired products if the reaction occurs in the presence of a small but effective amount of a reducing agent, such as hydrogen gas or a ferrous metal salt.

The present invention is a method of preparing an ether having the formula $[R-O]_n-R'$, wherein R is a substituted or unsubstituted alkyl group, R' is a substituted or unsubstituted aryl group and n is 1, 2, 3 or 4 comprising reacting an aryl compound containing at least one iodo substituent with an alkyl compound containing at least one hydroxy substituent in the presence of a basic compound and a copper catalyst and an effective amount of a reducing agent in order to reduce the formation of undesirable aryl by-products. The use of an effective amount of a reducing agent in the reaction allows the reaction to take place cheaply and efficiently, and as a result, this process can be used commercially in the ultimate production of less costly aromatic hydroxy compounds.

A number of iodo-substituted aryl compounds which contribute the -R' radical can be used in this reaction but it is preferred that either a monoiodo-, diiodo- or triiodo-substituted aryl compound be employed. The iodo-substituted aryl compound may also be further substituted by a number of suitable ether groups, such as amino, alkoxyl, hydroxyl and carboxyl ethers. Among the iodo aryl compounds suitable are p-diiodobenzene, m-diiodobenzene, 1,2-diiodobenzene, p-iodobenzoate, iodobenzene, iodonaphthalene, diiodonaphthalene, diiodo diphenyl ether, iodoaniline, iodotoluene, iodophenol, and diiodobiphenyl. Preferably p-diiodobenzene is used.

The alkyl compound containing at least one hydroxy substituent which contributes the R- radical is preferably an aliphatic alcohol or an alkyl glycol. Suitable hydroxyl alkyl compounds for the ether-forming reaction are simple alcohols, such as methanol, ethanol, etc., diols such as ethylene glycol or propylene glycol, and alcohols containing other functional groups. Branched alkyl compounds such as neophthyl glycol can be used. Preferably methanol is used.

The basic compound employed is preferably an alkali or alkaline earth metal hydroxide, or a salt of a weak organic acid. Illustrative of suitable alkali or alkaline earth metal hydroxides are sodium, lithium,

3

potassium and magnesium hydroxides. If the basic compound used is a salt of a weak organic acid, it is preferred that the organic acid be either carbonic acid or acetic acid. Suitable salts of these acids which can be employed as the base in the ether-forming reaction would thus include sodium acetate, sodium carbonate, lithium acetate, etc.

It has also been discovered that the choice of the basic compound used in conjunction with the reaction conditions of the ether-forming reaction can determine whether one or more of the iodine atoms in the iodoaromatic compound are replaced. By choosing the proper basic compound, selective replacement of one or more iodine atoms while one or more iodine atoms remain in place on the aromatic ring can be accomplished. Under different reaction conditions, the remaining iodine atoms can then be replaced with other functional groups. One example is the use of sodium acetate in the conversion of diiodobenzene to iodoanisole, which can be followed by carbonylation of the iodoanisole to the methyl ester of anisic acid, and cleavage of the ester and ether linkages to form hydroxybenzoic acid.

In general, in the ether-forming reaction of the present invention wherein the basic compound is an alkali or alkaline earth metal salt of a weak organic acid, and the aryl compound used is a diiodo-substituted aryl compound, the resulting ether will comprise a monoalkyl iodoaryl ether, when specific reaction conditions are present. To produce the monoalkyl iodoaryl ether, the reaction should be conducted at a temperature ranging from about 125°C to about 225°C, preferably from about 160°C to about 225°C, and the proportion of the diiodo aryl compound to the alkyl hydroxy compound should be about 1:1 to about 1:100 molar equivalents. Where the basic compound is an alkali or alkaline earth metal salt of a weak organic acid, and the aryl compound is a monoiodo-substituted aryl compound, the resulting ether will generally be a monoalkyl aryl ether.

Any reducing agent capable of lowering the oxidation state of the copper catalyst is suitable for use in the present invention. However, it is preferred that the reducing agent used be either hydrogen, in the form of hydrogen gas, or a ferrous metal salt, such as ferrous carbonate, ferrous chloride, or ferrous sulfate. In all instances, the reducing agent will be used in effective amounts, that is, an amount that minimizes undesirable aryl by-product formation. The actual amount of reducing agent employed will vary depending primarily on the particular reducing agent, reactants selected, and reaction conditions.

In a preferred mode of conducting the invention, the iodoaryl compound, hydroxyalkyl compound, base and copper catalyst are loaded into a rocking autoclave. The reducing agent is added before the reaction begins. If hydrogen gas is to be used as the reducing agent, the autoclave with the above ingredients can be closed and the hydrogen gas can be pumped in at room temperature. In a reaction mixture with about 10 grams of the iodoaromatic compound, one gram of copper catalyst, 7 grams of base, and about 100 mL of the alcohol, a charge of 100 psig of hydrogen gas should be sufficient to prevent degradation of the reactants, and to greatly minimize the production of undesirable aryl by-products. If a ferrous metal salt, such as ferrous carbonate, is added as the reducing agent, it should be introduced into the reaction mixture before the autoclave is closed. An amount of ferrous metal salt of about one gram added to the above ingredients should be effective in restricting the formation of unwanted by-products.

Once the ether-forming ingredients and the reducing agent are present in the closed autoclave, the autoclave is then heated so that the reaction is conducted at a temperature of about 160°C to about 225°C, and preferably at a temperature of from about 175°C to about 205°C. The reaction should also be conducted at a pressure of about 1-200 atm, and preferably from about 1 atm to about 100 atm pressure. The autoclave should be initially heated gradually for up to one hour until the reaction temperature is achieved, and the reaction temperature should be maintained for about 1-3 hours. After the reaction is completed, the autoclave should be cooled gradually for about one hour, after which the pressure can be vented, and the autoclave opened in order to collect the final product. This ether-forming reaction of the invention, using an effective amount of a reducing agent, is characterized by a high yield of desired reaction product and a minimum amount of the undesirable aryl by-products which would otherwise result from various degradative pathways.

The following examples are presented as illustrative of the present invention and should not be construed as limiting the invention in any way:

Example 1 - Prior Art

The following experiment was conducted to demonstrate the ether-forming reaction in the absence of the reducing agent used in this invention. A 300 mL Hastelloy C rocking autoclave was loaded with 10.0 g p-diiodobenzene (0.030 M), 1.0 g cuprous iodide, 7.4 g (0.09 M) anhydrous sodium acetate, and 100 mL methanol. The autoclave was closed and 100 psig nitrogen gas was added at room temperature. The autoclave was heated to 200°C over a 45-minute period and held at 200°C for two hours. The autoclave

EP 0 415 982 B1

was cooled over a one-hour period. The pressure was vented and the autoclave was opened. As determined by gas chromatographic methods of analysis, the liquid product contained 0.005 M iodobenzene, 0.007 M anisole, 0.005 M benzene, 0.002 M hydroquinone dimethyl ether, 0.009 M p-iodoanisole, 0.001 M p-diiodobenzene and trace amounts of several unidentified components. The iodine atoms originally present in the p-diiodobenzene are now present as sodium iodide. The formation of such large amounts of degradation products is unacceptable for a commercial process.

Example 2

Example 1 was repeated except that 100 psig hydrogen gas was used in place of 100 psig nitrogen gas. The liquid product contained 0.001 M anisole, 0.001 M iodobenzene, 0.004 M hydroquinone dimethyl ether, 0.020 M p-iodoanisole, and 0.004 M p-diiodobenzene. The iodine atoms originally present in the p-diiodobenzene are now present as sodium iodide. This demonstrates the ability of the hydrogen gas to prevent degradation during the reaction.

Example 3

Example 1 was repeated except that 1.0 g ferrous carbonate was added as the reducing agent. The liquid product contained 0.002 M anisole, 0.001 M iodobenzene, 0.006 M hydroquinone dimethyl ether, 0.018 M p-iodoanisole, and 0.002 M p-diiodobenzene.

Example 4

Example 3 was repeated by adding 1.0 g of other iron compounds in which iron was in the +3 valence state. Analyses of the liquid reaction products gave the following quantities of degradation products.

| Ferric Acetate | 0.002 M benzene 0.004 M anisole 0.005 M iodobenzene 0.002 M hydroquinone dimethyl ether 0.012 M p-iodoanisole 0.002 M p-diiodobenzene |
|---|---|
| Red Ferric Oxide | 0.001 M benzene 0.004 M anisole 0.004 M iodobenzene 0.007 M hydroquinone dimethyl ether 0.019 M p-iodoanisole 0.002 M p-diiodo benzene |
| Anhydrous Ferric Chloride | 0.002 M benzene 0.004 M anisole 0.006 M iodobenzene 0.003 M hydroquinone dimethyl ether 0.016 M p-iodoanisole 0.003 M p-diiodo benzene |

These results demonstrate that the function of the iron ion is as a reducing agent.

Example 5

Example 2 was repeated except that 3.6 g sodium hydroxide (0.09 M) was used in place of 7.4 g sodium acetate (0.09 M). The liquid product contained 0.027 M hydroquinone dimethyl ether, 0.001 M p-iodoanisole, and trace amounts of anisole, phenol, hydroquinone monomethyl ether, and p-diiodobenzene. Comparison of Example 5 with Example 2 demonstrates the effect of base strength on product composition. With sodium hydroxide, the predominant product is hydroquinone dimethyl ether, while under the same reaction conditions with the weaker base sodium acetate, the predominant product is p-iodoanisole.

5

### Example 6

Example 2 was repeated except that 10.0 g m-diiodobenzene was used in place of 10.0 g p-diiodobenzene. The liquid product contained 0.002 M anisole, 0.002 M iodobenzene, 0.011 M resorcinol dimethyl ether, 0.013 M 3-iodoanisole, and 0.001 M m-diiodobenzene.

### Example 7

Example 5 was repeated except that 10.0 g m-diiodobenzene was used in place of 10.0 g p-diiodobenzene. The liquid product contained 0.006 M anisole, and 0.023 M resorcinol dimethyl ether. Comparison of Example 6 with Example 7 again demonstrates the effect of base strength on reaction product composition.

### Example 8

As in Example 2, 11.4 g diiodonaphthalene (0.03 M, 60% 2,6-diiodo isomer and 40% 2,7-diiodo isomer), 1.0 g cuprous iodide, 3.6 g sodium hydroxide, 50 mL toluene, and 50 mL methanol were heated for four hours at 190°C under 100 psig $H_2$. The liquid product contained 0.025 M dimethoxy naphthalene and trace quantities of 2-methoxy naphthalene, 2-iodonaphthalene, and naphthalene.

### Example 9

As in Example 2, 10.0 g p,p'-diiododiphenyl ether (0.024 M), 1.0 g cuprous iodide, 7.4 g sodium acetate, 50 mL methanol, and 50 mL toluene were heated for four hours at 190°C under 100 psig $H_2$. The liquid product contained 0.001 M 4-methoxy diphenyl ether, 0.002 M 4-iodo diphenyl ether, 0.002 M dimethoxy diphenyl ether, 0.011 M iodomethoxy diphenyl ether, and 0.008 M p,p'-diiodo diphenyl ether.

### Example 10

As in Example 3, 7.9 g methyl p-iodo benzoate (0.03 M), 0.5 g cuprous iodide, 3.8 g sodium acetate, 1.0 g ferrous carbonate, and 100 mL methanol were reacted for two hours at 200°C. The liquid product contained 0.015 M methyl anisate, 0.006 M anisic acid, and 0.003 M methyl p-iodobenzoate.

### Example 11

As in Example 3, 8.0 g 1,2-diiodobenzene (0.024 M), 1.0 g cuprous iodide, 7.4 g sodium acetate, 1.0 g ferrous carbonate, and 100 mL methanol were reacted for two hours at 200°C. The liquid product contained 0.010 M catechol dimethyl ether, 0.011 M 2-iodoanisole, and traces of anisole, phenol, and iodobenzene.

### Example 12

As in Example 3, 6.6 g p-iodoaniline (0.03 M), 0.5 g cuprous iodide, 3.7 g sodium acetate, 1.0 g ferrous carbonate, and 100 mL methanol were reacted for two hours at 200°C. The liquid product contained 0.005 M aniline, 0.011 M p-anisidine, and 0.009 M p-iodoaniline.

### Example 13

As in Example 3, 7.6 g 1-iodonaphthalene (0.03 M), 0.5 g cuprous iodide, 1.8 g sodium hydroxide, 1.0 g ferrous carbonate, and 100 mL methanol were heated for two hours at 200°C. The liquid product contained 0.003 M naphthalene and 0.027 M 1-methoxy naphthalene.

### Example 14

As in Example 3, 6.5 g o-iodotoluene (0.03 M), 0.5 g cuprous iodide, 1.8 g sodium hydroxide, 1.0 g ferrous carbonate, and 100 mL methanol were heated for two hours at 200°C. The liquid product contained 0.004 M toluene and 0.026 M o-methoxy toluene.

### Example 15

As in Example 3, 7.0 g 2-iodoanisole (0.03 M), 0.5 g cuprous iodide, 1.8 g sodium hydroxide, 1.0 g ferrous carbonate, and 100 mL methanol were heated for two hours at 200°C. The liquid product contained 0.004 M anisole and 0.026 M catechol dimethyl ether.

### Example 16

As in Example 3, 6.6 g 2-iodoaniline (0.03 M), 0.5 g cuprous iodide, 3.7 g sodium acetate, 1.0 g ferrous carbonate, and 100 mL methanol were heated for two hours at 200°C. The liquid product contained 0.009 M aniline, 0.009 M o-anisidine, and 0.007 M 2-iodoaniline.

### Example 17

As in Example 3, 6.6 g m-iodophenol (0.03 M), 0.5 g cuprous iodide, 1.8 g sodium hydroxide, 1.0 g ferrous carbonate, and 100 mL methanol were heated for two hours at 200°C. The liquid product contained 0.002 M phenol, 0.024 M resorcinol monomethyl ether and 0.004 M m-iodophenol.

### Example 18

As in Example 2, 12.2 g p,p'-diiodobiphenyl (0.03 M), 1.0 g cuprous iodide, 3.6 g sodium hydroxide, and 100 mL methanol were heated for two hours at 200°C under 100 psig $H_2$. The liquid product contained 0.027 M p,p'-dimethoxy biphenyl and 0.002 M 4-methoxy biphenyl.

### Example 19

As in Example 2, 12.2 g p,p'-diiodobiphenyl (0.03 M), 9.2 g lithium acetate dihydrate (0.09 M), 1.0 g cuprous iodide and 100 mL methanol were heated for one hour at 200°C under 50 psig hydrogen gas. The liquid product contained 0.003 M p,p'-dimethoxybiphenyl, 0.014 M iodomethoxy biphenyl, and 0.013 M p,p'-diiodobiphenyl.

### Example 20

As in Example 2, 10.0 g m-diiodobenzene (0.03 M), 2.4 g sodium hydroxide, 1.0 g copper iodide, and 100 mL ethylene glycol were heated for two hours at 200°C under 100 psig $H_2$. The liquid product contained 0.027 M bis-hydroxyethyl resorcinol. This example demonstrates the use of an aliphatic dihydridic alcohol as the reacting alcohol.

### Example 21

As in Example 2, 6.1 g (0.03 M) iodobenzene, 1.8 g sodium hydroxide, 0.5 g cuprous iodide, 1.0 g ferrous carbonate, and 100 mL methanol were heated for two hours at 200°C under 100 psig $H_2$. The liquid product contained 0.028 M anisole and 0.001 M benzene.

## Claims

1. A method of preparing an ether having the formula [R-O]$_n$-R', wherein R is a substituted or unsubstituted alkyl group, R' is a substituted or unsubstituted aryl group and n is 1, 2, 3 or 4 comprising reacting an aryl compound containing at least one iodo substituent with an alkyl compound containing at least one hydroxy substituent in the presence of a basic compound, a copper catalyst and an effective amount of a reducing agent.

2. The method of Claim 1, wherein the reducing agent is selected from the group consisting of hydrogen and ferrous metal salts.

3. The method of Claim 2, wherein the reducing agent comprises a ferrous metal salt.

**4.** The method of Claim 3, wherein the ferrous salt is selected from the group consisting of ferrous carbonate, ferrous chloride and ferrous sulfate.

**5.** The method of Claim 1, wherein the aryl compound comprises a monoiodo-substituted aryl compound.

**6.** The method of Claim 1, wherein the aryl compound comprises a diiodo-substituted aryl compound.

**7.** The method of Claim 1, wherein said aryl compound is further substituted with a substituent selected from the group consisting of amino, alkoxyl, hydroxyl and carboxyalkyl ethers.

**8.** The method of Claim 1, wherein said alkyl compound containing at least one hydroxy substituent is selected from the group consisting of aliphatic alcohols and alkyl glycols.

**9.** The method of Claim 1, wherein said basic compound comprises an alkali or alkaline earth metal hydroxide.

**10.** The method of Claim 9, wherein the alkali or alkaline earth metal of the basic compound is selected from the group consisting of sodium, lithium, potassium and magnesium.

**11.** The method of Claim 1, wherein said basic compound comprises a salt of a weak organic acid.

**12.** The method of Claim 11, wherein said weak organic acid of the salts are selected from the group consisting of carbonic acid and acetic acid.

**13.** The method of Claim 1, wherein the reaction is conducted at a temperature of about 125°C to about 225°C; and

**14.** The method of Claim 13, wherein the reaction is conducted at a temperature of about 160°C to about 225°C.

**15.** The method of Claim 1, wherein the reaction is conducted at a pressure of about 1 atm. to about 200 atm.

**16.** The method of Claim 15, wherein the reaction is conducted at a pressure of about 1 atm. to about 100 atm.

**Patentansprüche**

**1.** Verfahren zur Herstellung eines Ethers der Formel [R-O]$_n$-R', worin R eine substituierte oder unsubstituierte Alkylgruppe darstellt, R' eine substituierte oder unsubstituierte Arylgruppe ist und n 1, 2, 3 oder 4 darstellt, bei dem man eine Arylverbindung mit mindestens einem Iod-Substituenten mit einer Alkylverbindung mit mindestens einem Hydroxy-Substituenten in Gegenwart einer basischen Verbindung, eines Kupfer-Katalysators sowie einer wirksamen Menge eines Reduktionsmittels miteinander umsetzt.

**2.** Verfahren nach Anspruch 1, bei dem das Reduktionsmittel ausgewählt ist aus der Gruppe bestehend aus Wasserstoff und Ferrometallsalzen.

**3.** Verfahren nach Anspruch 2, bei dem das Reduktionsmittel ein Ferrometallsalz ist.

**4.** Verfahren nach Anspruch 3, bei dem das Ferrosalz ausgewählt ist aus der Gruppe bestehend aus Ferrocarbonat, Ferrochlorid und Ferrosulfat.

**5.** Verfahren nach Anspruch 1, bei dem die Arylverbindung eine Monoiodo-substituierte Arylverbindung ist.

**6.** Verfahren nach Anspruch 1, bei dem die Arylverbindung eine Diiodo-substituierte Arylverbindung ist.

7. Verfahren nach Anspruch 1, bei dem die Arylverbindung weiterhin substituiert ist durch einen Substituenten, ausgewählt aus der Gruppe bestehend aus Amino-, Alkoxy-, Hydroxy- und Carboxyalkylethergruppen.

8. Verfahren nach Anspruch 1, bei dem die Alkylverbindung mindestens einen Hydroxy-Substituenten aufweist, der ausgewählt ist aus der Gruppe bestehend aus aliphatischen Alkoholen und Alkylgylkolen.

9. Verfahren nach Anspruch 1, bei dem die basische Verbindung ein Alkali- oder Erdalkalimetallhydroxid ist.

10. Verfahren nach Anspruch 9, bei dem das Alkali- oder Erdalkalimetall der basischen Verbindung ausgewählt ist aus der Gruppe bestehend aus Natrium, Lithium, Kalium und Magnesium.

11. Verfahren nach Anspruch 1, bei dem die basische Verbindung ein Salz einer schwachen organischen Säure ist.

12. Verfahren nach Anspruch 11, bei dem die schwache organische Säure des Salzes ausgewählt ist aus der Gruppe bestehend aus Kohlensäure und Essigsäure.

13. Verfahren nach Anspruch 1, bei dem die Reaktion bei einer Temperatur von etwa 125°C bis etwa 225°C durchgeführt wird.

14. Verfahren nach Anspruch 13, bei dem die Reaktion bei einer Temperatur von etwa 160°C bis etwa 225°C durchgeführt wird.

15. Verfahren nach Anspruch 1, bei dem die Reaktion bei einem Druck von etwa 1 atm. bis etwa 200 atm. durchgeführt wird.

16. Verfahren nach Anspruch 15, bei dem die Reaktion bei einem Druck von etwa 1 atm. bis etwa 100 atm. durchgeführt wird.

## Revendications

1. Procédé pour préparer un éther de formule $[R-O]_n-R'$, où R est un groupe alkyle substitué ou non, R' est un groupe aryle substitué ou non et n est 1, 2, 3 ou 4, procédé dans lequel on fait réagir un composé aryle comprenant au moins un substituant iodo avec un composé alkyle contenant au moins un substituant hydroxy en présence d'un composé basique, d'un catalyseur au cuivre et d'une quantité efficace d'agent réducteur.

2. Procédé selon la revendication 1, dans lequel l'agent réducteur est choisi dans le groupe comprenant l'hydrogène et les sels métalliques ferreux.

3. Procédé selon la revendication 2, dans lequel l'agent réducteur est un sel métallique ferreux.

4. Procédé selon la revendication 3, dans lequel le sel ferreux est choisi parmi le carbonate ferreux, le chlorure ferreux, et le sulfate ferreux.

5. Procédé selon la revendication 1, dans lequel le composé aryle est un composé aryle monoiodo substitué.

6. Procédé selon la revendication 1, dans lequel le composé aryle est un composé aryle diiodo substitué.

7. Procédé selon la revendication 1, dans lequel le composé aryle comprend de plus un substituant choisi parmi les groupes amino-, alkoxyl-, hydroxyl- et carboxyalkyl- éthers.

8. Procédé selon la revendication 1, dans lequel le composé alkyle contenant au moins un substituant hydroxy est choisi dans le groupe des alcools aliphatiques et des alkyl glycols.

**9.** Procédé selon la revendication 1, dans lequel le composé basique est un hydroxyde de métal alcalin ou alcalino-terreux.

**10.** Procédé selon la revendication 9, dans lequel le métal alcalin ou alcalino-terreux du composé basique est choisi parmi le sodium, le lithium, le potassium et le magnésium.

**11.** Procédé selon la revendication 1, dans lequel le composé basique est un sel d'acide organique faible.

**12.** Procédé selon la revendication 11, dans lequel la composante acide organique faible des sels est l'acide carbonique ou l'acide acétique.

**13.** Procédé selon la revendication 1, dans lequel la réaction se produit à une température comprise entre environ 125°C et environ 225°C.

**14.** Procédé selon la revendication 13, dans lequel la réaction se produit à une température comprise entre environ 160°C et environ 225°C.

**15.** Procédé selon la revendication 1, dans lequel la réaction se produit à une pression comprise entre environ 1 atm et environ 200 atm.

**16.** Procédé selon la revendication 15, dans lequel la réaction se produit à une pression comprise entre environ 1 atm et environ 100 atm.